# EUROPÄISCHE PATENTSCHRIFT

(11) **EP 1 276 878 B1**
(45) Veröffentlichungstag und Bekanntmachung des Hinweises auf die Patenterteilung: **08.12.2010**
(21) Anmeldenummer: 01915354.3
(22) Anmeldetag: 15.03.2001
(51) Int. Cl.: C12N 15/63, C12N 15/90, C12N 15/12, C12N 15/37, C12N 15/38, C12N 9/00, C12N 9/12, C12N 15/85, C12N 15/86, C12N 5/10, A61K 48/00, A61K 35/12, A61P 43/00

(54) **REVERSIBLE IMMORTALISIERUNG**
REVERSIBLE IMMORTALIZATION
IMMORTALISATION REVERSIBLE

(30) Priorität: 17.04.2000 DE 10019195
(43) Veröffentlichungstag der Anmeldung: 22.01.2003
(73) Patentinhaber: Medicyte GmbH, 69120 Heidelberg (DE)
(72) Erfinder: KÜPPER, Jan-Heiner, 47574 Goch (DE); KANDOLF, Reinhard, 72379 Hechingen (DE); KUHN, Anne, 72072 Tübingen (DE)
(74) Vertreter: Simandi, Claus
(86) Internationale Anmeldenummer: PCT/EP2001/002967
(87) Internationale Veröffentlichungsnummer: WO 2001/078757

(56) Entgegenhaltungen:
- WO-A-01/21790
- WO-A-93/01292
- KOBAYASHI N ET AL: "Prevention of acute liver failure in rats with reversibly immortalized human hepatocytes" SCIENCE, AAAS. LANCASTER, PA, US, Bd. 287, 18. Februar 2000 (2000-02-18), Seiten 1258-1262, XP002159501 ISSN: 0036-8075 in der Anmeldung erwähnt
- HOUGHTON A. ET AL.: "Immortalization of human marrow stromal cells by retroviral transduction with a temperature-sensitive oncogene: identification of bipotential precursor cells capable of directed differentiation to either an osteoblast or adiposcytte phenotype." BONE, Bd. 22, Nr. 1, Januar 1998 (1998-01), Seiten 7-16, XP001041404
- HICOK K.C. ET AL.: "Development and characterization of conditionally immortalized osteoblast precursor cell lines from human bone marrow stroma." J. BONE MINER. RES., Bd. 13, Nr. 2, Februar 1998 (1998-02), Seiten 205-217, XP001031498
- HAHN W C ET AL: "Creation of human tumour cells with defined genetic elements" NATURE, MACMILLAN JOURNALS LTD. LONDON, GB, Bd. 400, 27. Juli 1999 (1999-07-27), Seiten 464-468, XP002130651 ISSN: 0028-0836
- MACKENZIE K L ET AL: "EXTENDED LIPE SPAN OF HUMAN BONE MARROW ENDOTHELIAL CELLS TRANSFECTED WITH HUMAN TELOMERASE REVERSE TRANSCRIPTASE" BLOOD, W.B. SAUNDERS, PHILADELPHIA, VA, US, Bd. 92, Nr. 10, part 1, 1998, Seite 704A XP000930032 ISSN: 0006-4971
- TOMITA S ET AL: "AUTOLOGOUS TRANSPLANTATION OF BONE MARROW CELLS IMPROVES DAMAGED HEART FUNCTION" CIRCULATION, AMERICAN HEART ASSOCIATION, DALLAS, TX, US, Bd. 100, Nr. SUPPL 2, 1999, Seiten 247-256, XP002936395 ISSN: 0009-7322 in der Anmeldung erwähnt
- LEONG C.C. ET AL.: "Modulation of natural killer cell cytotoxicity in human cytomegalovirus infection: the role of endogenous Class I Major Histocompatibility Complex and viral Class I homolog" J. EXP. MEDICINE, Bd. 187, Nr. 10, 18. Mai 1998 (1998-05-18), Seiten 1681-1687, XP002183149
- MACHOLD R.P. ET AL.: "The HCMV gene products US11 and US2 differ in their ability to attack allelic forms of murine major histocompatibility complex MHC Class I heavy chains." J. EXP. MED., Bd. 185, Nr. 2, 20. Januar 1997 (1997-01-20), Seiten 363-366, XP002183150
- SHIN J.-S. ET AL.: "Monoclonal antibodies with various reactivity to p58 killer inhibitory receptors" HYBRIDOMA, Bd. 18, Nr. 6, Dezember 1999 (1999-12), Seiten 521-527, XP001041418
- DELGADO J.-P. ET AL.: "Long-term controlled immortalization of a primate hepatic progenitor cell line after Simian virus 40 T-Antigen gene transfer" ONCOGENE, Bd. 24, 20. Dezember 2004 (2004-12-20), Seiten 541-551,

## Beschreibung

Die vorliegende Erfindung beschäftigt sich mit Verfahren zur Gewinnung von Zellen, die z. B. in ein Organ transplantierbar sind. Allgemein betrifft die vorliegende Erfindung degenerative Erkrankungen, denen der Untergang definierter Zellpopulationen gemeinsam ist, sowie Transplantate und Arzneimittel zur Behandlung derartiger degenerativer Erkrankungen.

In den Industrieländern gibt es insbesondere durch die sich verändernde Alterspyramide eine Zunahme chronisch degenerativer Erkrankungen, die schwer oder noch gar nicht therapierbar sind.

Zu diesen Erkrankungen zählen unter anderem Herzmuskelerkrankungen, neurodegenerative Erkrankungen, Knochen- und Lebererkrankungen.

Die Schwere dieser Erkrankungen und die zunehmende Häufigkeit in der älter werdenden Bevölkerung ist mit einer immer kostspieliger werdenden medizinischen Behandlung sowie mit großen volkswirtschaftlichen Folgekosten verbunden. Dies trifft insbesondere für Erkrankungen des Herzmuskels zu, die als Folge von Koronargefäßverengungen, chronischer Herzmuskelentzündung oder mechanischer Überbeanspruchung auftreten können.

Diese Erkrankungen können zum einen zu einem akuten Myokardinfarkt führen, der in 1/3 der Fälle tödlich verläuft und oft jahrelang vorher durch Angina Pectoris-Anfälle angekündigt wird. Der Myokardinfarkt führt zu einem massiven nekrotischen bzw. apoptotischen Untergang von kontraktilen Herzmuskelzellen.
Das betroffene Areal vernarbt durch Vermehrung von Bindegewebszellen und Ablagerung von extrazellulärer Matrix, es kommt jedoch nicht zu einer Regeneration von Herzmuskelzellen.

Eine weitere wichtige Komplikation ist das sogenannte kongestive Herzversagen, das auf einer Herzhypertrophie beruht. Diese Hypertrophie stellt zunächst eine physiologisch sinnvolle Reaktion auf dauernd erhöhte Beanspruchung dar, führt aber ab einer kritischen Größe wieder zu einer Leistungsabnahme. Eine weitere Vergrößerung über diesen kritischen Punkt hinaus führt unweigerlich zum Herzversagen. Die heute einzig mögliche Therapie bei Herzhypertrophie ist die Herztransplantation.

Es ist bekannt, dass die Herzhypertrophie auf einer Vergrößerung einzelner Zelltypen sowie auf einem Umbau kontraktilen Herzmuskelgewebes durch Bindegewebe basiert. Die hochgradig differenzierten Herzmuskelzellen haben ihre Fähigkeit zur Regeneration durch Zellteilung verloren. Es kommt sogar durch verschiedene biochemische und mechanische Reize zur Auslösung von programmiertem Zelltod (Apoptose) in Herzmuskelzellen.

Insgesamt sind sowohl die dekompensierte Herzhypertrophie als auch der Myokardinfarkt durch einen großen Verlust an kontraktilen Herzmuskelzellen gekennzeichnet.

Auch bei neurodegenerativen Erkrankungen kann der Verlust definierter Zelltypen den Krankheitsverlauf bestimmen. So kommt es bei Morbus Parkinson, der häufigsten neurologischen Erkrankung im fortgeschrittenen Lebensalter, zu einer kontinuierlichen Abnahme der Dopamin-produzierenden Zellen in der Substantia nigra. Auch hier handelt es sich sehr wahrscheinlich um apoptotischen Zelltod.

Es konnte nachgewiesen werden, dass die gezielte Transplantation fetaler dopaminerger Zellen in diese Substantia nigra schwerste klinische Manifestationen von Parkinson ganz erheblich verbessern kann.

Die altersbedingte Osteoporose, eine fortschreitende Skeletterkrankung, insbesondere in der weiblichen Bevölkerung, ist durch den Verlust von Knochensubstanz gekennzeichnet, dem ein Rückgang in der Zahl und der Aktivität knochenbildender Zellen (Osteoblasten, Osteozyten) vorausgeht.

Fortschreitende Leberschädigung entsteht durch Alkoholabusus, chronische Entzündungen oder stoffwechselbedingt bzw. als Folge kardiovaskulärer Erkrankungen und zeichnet sich durch den Verlust von physiologisch aktiven Leberparenchymzellen (Hepatozyten) aus.

Für keine der insoweit lediglich beispielhaft angesprochenen Erkrankungen gibt es zur Zeit eine kurative Therapie.

Die bei Herzmuskelerkrankungen verschriebenen Medikamente können zwar Herzfunktionen wie Kontraktilität und Reizleitung positiv beeinflussen, aber kein verlorengegangenes Herzmuskelgewebe ersetzen. Oft wirken Medikamente auch nur sypmtomatisch, indem sie beispielsweise das Gewebswasser bei portaler Hypertension nach Leberzirrhose entziehen, oder Schmerzen bei Osteoporose lindern.

Bei fortgeschrittenen Herzmuskelerkrankungen oder bei dekompensierter Leberzirrhose ist die Organ-Transplantation die einzige verbleibende Möglichkeit. Diese ist aber limitiert durch rückläufige Zahlen von Spenderorganen und zudem mit hohen Behandlungskosten verbunden. Darüber hinaus besteht bei der Organtransplantation das immanente Problem der Gewebeabstoßung, so dass die Patienten lebenslang mit Immunsuppressiva behandelt werden müssen. Die größten dabei entstehenden Komplikationen sind die opportunistischen Infektionen mit Viren, Bakterien und Pilzen, die nicht selten tödlich verlaufen.

Bei neurodegenerativen Erkrankungen wie Morbus Parkinson gibt es darüber hinaus nicht einmal die Möglichkeit einer Organ-Transplantation. Bei dieser Krankheit hat man aber schon zum Teil mit gutem Erfolg versucht, dopaminerge Zellen aus den Gehirnen abgetriebener Feten zu transplantieren.

In der Literatur gibt es bereits vereinzelte Hinweise, dass die insoweit beispielhaft angesprochenen degenerativen Erkrankungen durch die Transplantation von Zellen behandelt werden können.

Kobayashi et al."Prevention of acute liver failure in rats with reversibly immortalized human hepatocytes", Science, Band 287, Seiten 1258-1262 beschreiben einen retroviralen Vektor, mit dem primäre humane Hepatozyten infiziert wurden. Die in vitro vermehrten Hepatozyten wurden in die Milz von hepatektomierten Ratten injiziert, und es konnte gezeigt werden, dass nach der Injektion dieser Zellen wichtige Leberfunktionen unterstützt wurden. Das dort vorgeschlagene Verfahren soll die Zeit bis zur Transplantation oder spontanen Regenerierung der Leber überbrücken. Prinzipiell zeigt diese Veröffentlichung, dass durch Zelltransplantation klinisch erwünschte Effekte erzeugt werden können.

Der in dem bekannten Verfahren verwendete retrovirale Vektor enthält einen Genbereich mit einem Resistenzgen, einem Suizidgen und einem Transformationsgen, der von zwei LoxP-Stellen flankiert wird. An die stromabwärts gelegene LoxP-Stelle schliesst sich ein weiteres Resistenzgen an, dem jedoch ein Initiierungskodon fehlt, so dass dieses zweite Resistenzgen nicht translatiert werden kann. Durch das Transformationsgen, in diesem Falle das Tumorantigen von SV40, werden die ruhenden Hepatozyten wieder in die Proliferation getrieben, wobei das erste Resistenzgen eine Resistenz gegen Hygromycin und das Suizidgen eine Empfindlichkeit gegenüber Ganciclovir bewirkt. Auf diese Weise kann auf erfolgreiche Transformation selektioniert werden.

Eine der so selektionierten Zellinien wurde offenbar immortal und konnte in einem entsprechenden Medium expandiert werden.
Nach der Expansion wurden die Zellen mit einem replikationsinkompetenten rekombinanten Adenovirus transduziert, der die Cre-Rekombinase exprimierte. Durch die Cre-Rekombinase wurde der zwischen den beiden LoxP-Stellen befindliche Genbereich exzisiert, so dass das Onkogen SV40 T-Ag nicht länger exprimiert wurde; zum Cre-Lox-System siehe z.B. Rajewsky et al., "Conditional gene targeting", J. Clin. Invest. Band 98, Seiten 600-603. Durch die Exzision mit der Cre-Rekombinase erfolgte eine intramolekulare Rekombination, so dass jetzt das außerhalb des von den LoxP-Stellen flankierten Genbereiches liegende Resistenzgen unmittelbar hinter einem Startkodon zu liegen kam und folglich eine Resistenz vermitteln konnte. Auf diese Weise konnte der Erfolg der Exzision durch Selektion auf Resistenz gegen hier G418 (Neomycin Resistenzgen) verifiziert werden.

In der genannten Veröffentlichung wird dieser Vorgang zwar als reversible Immortalisierung beschrieben, nach Erkenntnis der Erfinder der vorliegenden Anmeldung handelt es sich dabei jedoch um eine reversible Transformation, die bei einem Klon durch spontane Mutation zu immortalen Zellen geführt hat. Es ist nämlich bekannt, dass mit dem Transformationsprinzip durch SV40T-Ag bei Zellen lediglich eine "extended life span" erzeugt werden kann; siehe hierzu z. B. Chiu und Harley, "Replicative senescence and cell immortality : the role of telomeres and telomerase", Proc. Soc. Exp. Biol. Med. Band 214, Seiten 99-106. Chiu und Harley geben einen Kurzüberblick über die Telomer Hypothese, die davon ausgeht, dass die Telomer-Länge als methodische Uhr für die Steuerung der replikativen Lebensdauer von Zellen dient. Sie berichten, dass die Expression der Telomerase die Telomer-Länge stabilisiert und eine kontinuierliche Replikation oder Zell-Immortalität ermöglicht. Sie beschreiben ebenfalls die therapeutischen Möglichkeiten, die mit dieser Hypothese verbunden sind, und diskutieren, ob durch die Aktivierung der Telomerase-Expression in vivo oder ex vivo das replikative Potential von Zellen erhöht werden kann. Sie berichten ferner, dass in vielen Tumorzellen eine Telomerase-Aktivität vorhanden ist, die durch eine spontane Mutation hervorgerufen wurde. Ausgehend von dem Artikel von Chiu und Harley ist davon auszugehen, dass die von Kobayashi et al. a. a. O. berichtete immortale Zellinie nach der Transformation mit dem Onkogen durch eine spontane Mutation entstanden ist. Dies bedeutet jedoch, dass das von Kobayashi et al. a. a. O. beschriebene Verfahren nicht reproduzierbar und damit kommerziell nicht sinnvoll einsetzbar ist.

Wenn humane primäre Zellen in Kultur genommen werden, so teilen sich diese je nach Alter des Spenders noch 20-60 mal und gehen dann in die Senescenz. Durch den bei jeder Teilung entstehenden Telomerverlust wird ein Zellteilungsstop induziert, der durch die Transformation solcher Zellen mit einem Onkogen umgangen werden kann. Diese Zellen können sich über diese erste Krise hinaus weiter teilen. Dann entsteht jedoch irgendwann eine zweite Krise, da der bei jeder Zellteilung entstehende Telomerverlust zu genetischer Instabilität führt. Diese zweite Krise ist für fast alle mit einem Tumor-Antigen transformierten Zellen tödlich. In weniger als 10⁻⁶ der Fälle entsteht jedoch durch spontane Mutation eine Aktivierung der Telomerase, so dass der Telomerverlust ab dann kompensiert wird. Dies nennt man spontane Immortalisierung, wie sie auch bei Kobayashi et al. a. a. O. stattgefunden haben muss. Diese Aktivierung der Telomerase kann auch nicht gezielt wieder abgeschaltet werden, so dass selbst nach Exzision des Tumorantigens die Gefahr bleibt, dass diese Zellen durch ein weiteres Mutationsereignis, das erneut eine Wachstumsinduktion bewirkt, zu Krebszellen entarten.

Das insoweit von Kobayashi et al. beschriebene Verfahren weist also eine ganze Reihe von Nachteilen auf. Zum einen hängt der Erfolg des Verfahrens davon ab, dass eine spontane Aktivierung der Telomerase erfolgt. Dies bedeutet jedoch, dass das Verfahren nicht reproduzierbar und somit kommerziell nur sehr begrenzt verwendbar ist. Zum anderen kann die Telomerase-Aktivität nicht gezielt wieder abgeschaltet werden, so dass die Zellen auch nach Exzision des Tumorantigens zu Krebszellen entarten können. Ein weiterer Nachteil besteht darin, dass auch nach der Exzision ein Teil des retroviralen Vektors in den expandierten Zellen aktiv bleibt und das Resistenzgen exprimiert, das zur Selektion auf erfolgreiche Exzision verwendet wird. Dieses zusätzliche genetische Material steht einer Transplantation der so behandelten Zellen in humane Organe jedoch entgegen.

Während Kobayashi et al. sich mit der Herstellung von transplantierbaren Zellen aus terminal differenzierten Ausgangszellen beschäftigen, ist es auch bereits bekannt, autologe Stromazellen aus dem Knochenmark in das Herz zu transplantieren und dadurch die Herzfunktion zu verbessern ; siehe Tomita et al.,"Autologous transplantation of bone marrow cells improves damaged heart function", Circulation Band 100, Seiten 11247- 11256. Die Kultivierung der Knochenmarkszellen erfolgte unter Zugabe der Differenzierungssubstanz 5'-Azacytidin, wodurch eine Differenzierung der Knochenmarkszellen zu kardiomyogenen Zellen erfolgte.

Wegen der limitierten replikativen Kapazität der autologen Knochenmark-Stromazellen kann auf diese Weise die beim Menschen benötigte Menge an regenerativen Zellen jedoch nicht reproduziert werden. Da Herzerkrankungen Alterserkrankungen sind, sind auch die meisten autologen Spender relativ alt, so dass sich die dem Patienten entnommenen autologen Knochenmarkszellen vielleicht noch zwanzig mal teilen können. Theoretisch kann man somit aus einer Zelle nur noch ca. 10⁶ Zellen herstellen, was weniger als 0,02 % des linken Ventrikels entspricht. Das von Tomita et al. a. a. O. beschriebene Verfahren ist also für klinische Anwendungen nicht geeignet.

Auch Makino et al.,"Cardiomyocytes can be generated from marrow stromal cells in vitro", J. Clin. Invest. Band 103, Seiten 697-705 beschäftigen sich mit der Differenzierung von Knochenmarkszellen in Herzmuskelzellen durch Zugabe von 5'-Azacytidin.
Durch eine häufige Subkultivierung der Stromazellen für mehr als vier Monate wurde eine immortalisierte Zellinie erhalten, die als Ausgangsmaterial für die Differenzierung in Herzmuskelzellen diente. Dieses Verfahren ist mit den gleichen Nachteilen behaftet wie das von Kobayashi et al. a. a. O beschriebene Verfahren, es ist weder reproduzierbar noch klinisch tolerierbar.

Vor diesem Hintergrund liegt der vorliegenden Erfindung die Aufgabe zugrunde, immunologisch und klinisch unbedenkliche Zellen auf kostengünstige Weise bereitzustellen, die z. B. für eine lokale Geweberegeneration verwendet werden können.

Erfindungsgemäß wird diese Aufgabe gelöst durch ein Verfahren zur Gewinnung von Zellen, mit den Schritten: (a) Bereitstellen von organbezogenen Zellen, (b) Immortalisieren der organbezogenen Zellen durch Transfer eines erfindungsgemäßen Genkomplexes, (c) Expansion der immortalisierten Zellen aus (b) und Reversion der Immortalisierung der expandierten Zellen. Als organbezogene Zellen können dabei multipotente Stammzellen, vorzugsweise mesenchymale Stromazellen oder aber ruhende, terminale differenzierte Ausgangszellen des Organs verwendet werden.

Durch die reversible Immortalisierung sind die so hergestellten Zellen klinisch unbedenklich. Die Zellen können außerdem in unbegrenzter Zahl hergestellt werden.

Wenn multipotente Stammzellen als organbezogene Zellen verwendet werden, erfolgt die Expansion der immortalisierten Stammzellen unter Zugabe zumindest einer Differenzierungssubstanz, die eine Differenzierung der Stammzellen zu organspezifischen Zellen fördert.

Werden dagegen terminal differenzierte Ausgangszellen eingesetzt, so werden diese im Zusammenhang mit der Immortalisierung zusätzlich transformiert, so dass sie sich expandieren lassen.

Mit den so hergestellten Zellen kann z. B. eine Transplantation in einem Herzinfarktareal durchgeführt werden, wodurch gleichzeitig das Risiko eines kongestiven Herzversagens sowie eines sekundären tödliche Herzinfarktes erheblich vermindert wird.
Das Verfahren eignet sich auch zur Gewinnung regenerativer Knochen- und Knorpelzellen, die bei Knochen- und Knorpeltraumata sowie bei chronischer Knochendegeneration (Osteoporose) eingesetzt werden können. Mit dem Verfahren können auch Leber parenchymzellen für die Leberregeneration sowie dopaminerge Zellen zur Behandlung des Morbus Parkinson hergestellt werden.

Das erfindungsgemäße Verfahren ermöglicht die Produktion beliebiger Mengen primärer Zellen für die extrakorporale Herstellung von Gewebe. Nach dem neuen Verfahren produzierte Endothelzellen oder Glatte Muskelzellen können auf einer Matrix, vorzugsweise einer Biomatrix beispielsweise aus Collagen oder Fibronektin angesiedelt werden, um Herz- oder Venenklappen zu erzeugen.

Bei der Herstellung von z. B. Muskelzellen, vorzugsweise von Herzmuskelzellen, sowie von Knochenzellen, ist es bevorzugt, wenn bei der Expansion der immortalisierten Stammzellen eine Differenzierungssubstanz zugegeben wird, die ausgewählt ist aus der Gruppe : Dexamethason, 5'-Azacytidin, Trichstatin A, all-trans-Retinsäure und Amphotericin B. Besonders bevorzugt ist es dabei, wenn zumindest zwei, vorzugsweise vier dieser Differenzierungssubstanzen bei der Expansion verwendet werden.

Obwohl eine Differenzierung von Stammzellen zu Herzmuskelzellen durch Zugabe von 5'-Azacytidin induziert wird, kann die Differenzierung durch Zugabe zumindest einer weiteren Differenzierungssubstanz verbessert werden. Besonders geeignet ist dabei eine Kombination von 5'-Azacytidin und Trichstatin A, was nach Erkenntnis der Erfinder der vorliegenden Anmeldung synergistisch wirkt. Die Differenzierung kann durch die weitere Zugabe von all-trans-Retinsäure und Amphotericin B weiter optimiert werden.

Neben der in der Kombination aus mehreren Differenzierungssubstanzen liegenden synergistischen Wirkung liegt ein weiterer Vorteil darin, dass die nach Erkenntnis der Erfinder vorliegende mutagene Wirkung von 5'-Azacytidin erheblich reduziert oder sogar aufgehoben wird.

Auf diese Weise ist eine unbedenkliche klinische Anwendung der so aus Stammzellen gewonnenen Herzmuskelzellen möglich.

Durch die Zugabe der Differenzierungssubstanz Dexamethason werden die reversibel immortalisierten Stammzellen zu Knochen-und Knorpelzellen differenziert. Auch hier kann ein synergistischer Effekt durch die weitere Zugabe der Differenzierungssubstanzen 5'-Azacytidin, Trichstatin A, all-trans-Retinsäure und Amphotericin B erzielt werden.

Zu der Differenzierungssubstanz Dexamethason sei noch erwähnt, dass Conget und Minguell "Phenotypical and functional properties of human bone marrow mesenchymal progenitor cells", J. Cell Physiol. Band 181, Seiten 67-73 bereits beschrieben haben, dass sich osteogene Zellen aus mesenchymalen Stromazellen durch Dexamethason-Behandlung gewinnen lassen.

Als organbezogene Zellen können dabei sowohl autologe als auch allogene Zellen eingesetzt werden.

Während der Vorteil der autologen Zellen in der Immuntoleranz liegt, weisen die allogenen Zellen den Vorteil auf, dass sie quasi jederzeit in unbegrenzter Zahl zur Verfügung stehen. Bei den allogenen Zellen wird jedoch erfindungsgemäß eine Immuntoleranz erzeugt, um die Host versus Graft Reaktion (HVGR) zu reduzieren.

Zur Behandlung eines Patienten können dann folglich zunächst aus allogenen Zellen hergestellte transplantierbare Zellen verwendet werden, während parallel aus autologen Zellen des Patienten weitere transplantierbare Zellen hergestellt werden. Wenn dann genügend autologe transplantierbare Zellen zur Verfügung stehen, werden nur noch diese transplantiert, so dass die Immuntoleranz dann kein Problem mehr darstellt.

Erfindungsgemäß wird für die reversible Immortalisierung der Zellen ein Genkomplex verwendet, mit einem immortalisierenden Genbereich, der zumindest ein Resistenzgen, ein Telomerase-Gen und ein Suizidgen aufweist, mit zwei den Genbereich flankierenden Sequenzen, die als Erkennungsstelle für homologe intramolekulare Rekombination fungieren, und zwei, dem immortalisierenden Genbereich und den Sequenzen vorangestellten, jeweils flankierende Promotoren.

Wenn dieser Genkomplex in eine organbezogene Zelle eingebracht wird, so kann zunächst mittels des Resistenzgenes auf den erfolgreichen Transfer selektioniert werden.

Durch das Telomerase-Gen ist, wird bei der Expansion jetzt Telomerverlust vermieden, so dass die Zellen unbeschränkt replikationsfähig sind, sofern es sich bei den organbezogenen Zellen um Stammzellen handelt, die zur Proliferation fähig sind.

Wenn als organbezogene Zellen dagegen terminal differenzierte Ausgangszellen verwendet werden, enthält der Genkomplex zusätzlich ein Transformationsgen, das vorzugsweise das Tumor-Antigen von SV40, also ein Onkogen, ist. Auf diese Weise können auch ruhende Zellen immortalisiert und zur Herstellung von transplantierbaren Zellen verwendet werden.

Durch die homologe, intramolekulare Rekombination wird nach erfolgreicher Expansion der Genbereich, der das Resistenzgen, das Telomerase-Gen und das Suizidgen enthält, aus dem Genkomplex exzisiert, so dass die Immortalisierung zuverlässig aufgehoben wird. Damit besteht bei derart hergestellten Zellen keine über das übliche Maß erhöhte Gefahr, dass sie nach der Transplantation zu Krebszellen entarten.

Vorzugsweise sind die flankierenden Sequenzen dabei LoxP Stellen, wobei zur intramolekularen Rekombination Cre-Rekombinase verwendet wird. Die Immortalisierung der expandierten Zellen kann dabei zu einem gewünschten Zeitpunkt dadurch wieder rückgängig gemacht werden, dass die Zellen z. B. mit einem rekombinanten Virus, bspw. einem rekombinanten Adenovirus infiziert werden, das die Cre-Rekombinase exprimiert, oder dass die Cre Rekombinase als zellgängiges rekombinantes Fusionsprotein, bspw. als Fusionsprotein mit dem Voyager Protein VP22, verabreicht wird.

Vorzugsweise wird dabei durch das Suizidgen auf erfolgreiche Exzision selektioniert. Das Suizidgen ist dabei vorzugsweise das Thymidinkinase-Gen von Hepatitis Simplex-Virus (HSV). Nach der Exzision des immortalisierenden Genbereiches ist auch das Suizidgen aus dem transferierten Genkomplex entfernt, so dass diese Zellen nicht mehr sensitiv gegenüber Ganciclovir sind. Bei den Zellen, bei denen die Exzision nicht stattgefunden hat, ist die Sensitivität gegenüber Ganciclovir jedoch noch vorhanden, so dass sie abgetötet werden.

Ein großer Vorteil bei dem insoweit beschriebenen Verfahren ist darin zu sehen, dass nach der Reversion der Immortalisierung der Genkomplex keine exprimierbaren Gene mehr enthält, so dass die Transplantation derartiger Zellen klinisch völlig unbedenklich ist.

Miller und Sedmak,"Viral effects on antigen processing", Curr. Opin. Immunol. Band 11, Seiten 94-99 konnten zeigen, dass diese Genprodukte des humanen Cytomegalievirus (CMV) eine weitgehende Degradation von intrazellulären MHC 1-Molekülen bewirkt.

Da eine Verringerung oder vollständige Blockade der MHC I Präsentation auf Zellen zu einer Aktivierung von Natürlichen Killerzellen führt, wird die Aktivität dieser Natürlichen Killerzellen durch Expression des CMV-Gens UL18 inhibiert.

Die MHC I-Inaktivierung kann auch durch andere virale Gene, z. B. das HSV-Gen ICP47, die CMV-Gene US6 und US3, die Adenovirusgene E3-19K sowie E6 oder das HIV NEF-Gen erfolgen. Ferner ist auch der direkte Knock-out eines MHC I-Bestandteiles, z. B. des beta-2-Microglobulingens, möglich. Ferner kann auch intrazellulär ein rekombinanter single chain Antikörper exprimiert werden, der die MHC-Präsentation auf der Zelloberfläche blockiert.

Natürliche Killerzellen können auch über einen monoklonalen Antikörper inaktiviert werden, der die sogenannten inhibitory receptors der Natürlichen Killerzellen erkennt und die NK vermittelte Zell-Lyse blockiert. Dieser monoklonale Antikörper wird erfindungsgemäß auch als rekombinanter single chain Antikörper in der Membran der allogenen Spenderzelle verankert und kann dadurch angreifende NK-Zellen abwehren.

Mit Hilfe des neuen Verfahrens und ggf. unter Verwendung der neuen Genkomplexe hergestellte Zellen sind ebenfalls Gegenstand der vorliegenden Erfindung. Diese Zellen können erfindungsgemäß zur Herstellung eines Transplantates für die Regeneration eines Organes oder zur Herstellung eines Arzneimittels zur Behandlung chronischer Erkrankungen verwendet werden.

Vor diesem Hintergrund betrifft die vorliegende Erfindung ebenfalls ein Arzneimittel, das die erfindungsgemäß hergestellten Zellen in therapeutisch wirksamer Menge enthält, sowie ein Transplantat, das diese Zellen enthält.
Ferner betrifft die vorliegende Erfindung die Verwendung der Zellen zur Regeneration eines Organes.

Schließlich betrifft die Erfindung noch ein Plasmid, einen viralen Vektor bzw. ein Kit mit dem Genkomplex zur reversiblen Immortalisierung.

Das erfindungsgemäße Plasmid bzw. der erfindungsgemäße virale Vektor werden für den Transfer der Genkomplexe in die organspezifischen Zellen verwendet, um diese zu immortalisieren und ggf. zu transformieren.

Der erfindungsgemäße Kit kann neben den Genkomplexen die weiter erforderlichen Substanzen und Materialien, z. B. für die Cre Rekombinase codierende rekombinante Adenoviren, enthalten.

Mit Hilfe des Kits können dann allogene oder autologe Spenderzellen reversibel immortalisiert und expandiert werden, bevor sie dann zur Organregeneration transplantiert werden.

Weitere Vorteile ergeben sich aus der Beschreibung und der beigefügten Zeichnung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

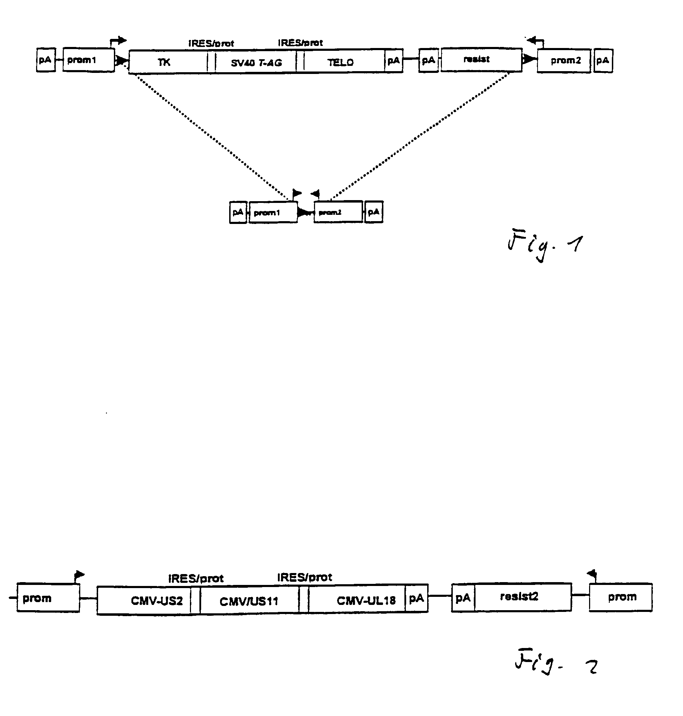

Die Erfindung wird jetzt anhand von Ausführungsbeispielen und der beigefügten Zeichnung erläutert. Es zeigen: Fig. 1 einen Genkomplex zur reversiblen Immortalisierung von Zellen, in schematischer Darstellung ; und Fig. 2 einen Genkomplex zur Immunmodulation von Zellen, in schematischer Darstellung.

### Beispiel 1 : Bereitstellen von organbezogenen Zellen

Als organbezogene Zellen können multipotente Stammzellen verwendet werden, die bei der Expansion noch in organspezifische Zellen differenziert werden müssen, oder bereits differenzierte Ausgangszellen des jeweiligen Organes.

Weiter muss zwischen autologen Zellen des jeweiligen Patienten oder allogenen Zellen eines Spenders unterschieden werden. Autologe Zellen können ohne weitere immunmodulatorische Behandlung verwendet werden, während allogene Zellen mit immunmodulatorischen Genen stabil transfiziert werden. Der Vorteil von allogenen Zellen besteht darin, dass sich diese in großer Zahl herstellen lassen und für viele Empfänger sofort verwendbar sind, wobei das Risiko einer Host versus Graft Reaktion (HVGR) wegen der immunmodulatorischen Behandlung (siehe Beispiel 2) sehr gering ist. Bei autologen Zellen besteht ein Vorteil darin, dass kein HVGR-Risiko besteht.

Als Stammzellen werden mesenchymale Stromazellen des Knochenmarks verwendet. Diese sind in der Lage, sich zu Osteoblasten, Myoblasten, Adipocyten sowie weiteren Zelltypen zu differenzieren. In der Klinik wird Knochenmark routinemäßig unter OP Bedingungen für die allogene Knochenmarktransplantation gewonnen. Dabei werden jedoch nur die hämatopoetischen Stammzellen benötigt, während die hier interessanten mesenchymalen Stammzellen als Nebenprodukt entstehen.

Anderseits können mesenchymale Stammzellen auch aus peripherem Blut gewonnen werden.

Die so gewonnenen Stammzellen werden in konventionellen Zellkulturschalen ausgesät und in alpha-MEM oder IDEM-Medium mit 10 % FKS, sowie Antibiotika wie Penicillin, Streptomycin oder Amphotericin B kultiviert.

### Hepatozyten der Leber werden direkt als Primärkultur angelegt.

Dopaminerge Ausgangszellen werden im Rahmen einer Organspende entnommen. Herzmuskelzellen können sowohl als Stammzellen aus Knochenmark als auch als Ausgangszellen im Rahmen einer Herzmuskel-Biopsie für die Immortalisierung gewonnen werden.

### Beispiel 2 : Immortalisierung, Transformation und Immunmodulation

Die insoweit gewonnenen, organbezogenen Zellen werden jetzt reversibel immortalisiert, wobei die terminal differenzierten Ausgangszellen auch noch transformiert werden müssen. Bei allogenen organbezogenen Zellen ist ferner noch eine Immunmodulation erforderlich.

Der in Fig. 1 dargestellte Genkomplex dient der reversiblen Immortalisierung der organbezogenen Zellen. Der in Fig. 2 dargestellte Genkomplex dient dagegen zur Immunmodulation von allogenen Spenderzellen.

Beide Genkomplexe können über Plasmidtransfektion oder virale Transduktion in die organbezogenen Zielzellen eingebracht werden. Auf die erfolgreiche Übertragung der Genkomplexe kann mit dem jeweiligen Resistenzgen selektioniert werden.

Das Resistenzgen in dem Genkomplex aus Fig. 1 ist z. B. das Neomycin-Gen, das eine Resistenz gegenüber G418 vermittelt. Das Resistenzgen in Fig. 2 ist z. B. das Hygromycin-Gen, das eine Resistenz gegenüber Hygromycin vermittelt.

Der Genkomplex in Fig. 1 enthält als Transformationsgen das large tumor Antigen von SV40 sowie als Immortalisierungsgen das Telomerase-Gen. Das Transformationsgen wird dabei nur für ruhende terminal differenzierte Ausgangszellen benötigt, für proliferierende Stammzellen reicht das Immortalisierungsgen für die Telomerase.

Ferner enthält der Genkomplex aus Fig. 1 ein Suizidgen, nämlich das Thymidinkinase-Gen, das eine Sensitivität gegenüber Ganciclovir vermittelt.

Der immortalisierende Genbereich aus Suizidgen TK, Transformationsgen SV40T-Ag und Telomerase-Gen TELO sowie dem ersten Resistenzgen resist wird flankiert von zwei LoxP-Stellen. Über das Enzym Cre-Rekombinase des Bakteriophagen P1 kann an zwei identischen LoxP-Sequenzen eine homologe Rekombination herbeigeführt werden. Eine LoxP-Stelle ist eine 34 Basenpaare lange DNA-Sequenz, die aus zwei 13 Basenpaaren langen inverted repeats besteht, welche von einer 8 Basenpaaren langen nichtpalindromischen Sequenz unterbrochen ist. Wenn zwei LoxP Stellen in derselben Orientierung auf einem linearen DNA Molekül plaziert sind, wird durch die Cre-Rekombinase eine intramolekulare Rekombination bewerkstelligt, die zur Exzision der zwischen den beiden LoxP-Stellen befindlichen Sequenz führt. Dieses ist hochspezifisch und sehr effizient, da die exzisierte DNA durch Degradation aus dem Gleichgewicht entfernt wird.

Die Expression der hintereinandergeschalteten Gene kann durch Genfusion, durch interne Translation mit einer IRES (internal ribosomal entry site) oder durch interne Proteolyse (prot) bewerkstelligt werden. Bei der letzten Möglichkeit befindet sich zwischen den Genen eine Protease, die sich selbst in cis herausschneidet und somit die einzelnen Genfunktionen separiert. Als interne Protease kann die 2A-Protease von FMDV (Foot and Mouth Disease Virus) verwendet werden.

Nach erfolgreichem Transfer des Genkomplexes aus Fig. 1 in die organbezogenen Zellen aus Beispiel 1 können diese beliebig expandiert werden. Handelt es sich bei den organbezogenen Zellen um multipotente Stammzellen, erfolgt die Expansion unter Zugabe zumindest einer Differenzierungssubstanz, die eine Differenzierung der Stammzellen zu organspezifischen Zellen fördert, wie es in Beispiel 3 weiter unten beschrieben wird.

Wenn die organbezogenen Zellen allogene Zellen eines Spenders sind, muss zusätzlich zu dem Genkomplex aus Fig. 1 auch der Genkomplex aus Fig. 2 übertragen werden, um eine Immuntoleranz zu erzielen.

Dies erfolgt durch die Expression der CMV-Gene US2 und US11, die die Besetzung der Zelloberflächen der organbezogenen Zellen mit MHC 1-Molekülen verhindert. Die Verringerung oder vollständige Blockade der MHC 1-Präsentation verhindert zum einen zwar die Lyse der körperfremden Spenderzellen durch zytotoxische T Lymphozyten, führt aber andererseits automatisch zu einer Aktivierung von Natürlichen Killerzellen.

Durch die Expression des CMV-Genes UL18 wird über einen noch nicht genau verstandenen Mechanismus jetzt erfindungsgemäss die Aktivität der Natürlichen Killerzellen inhibiert.

In Fig. 1 und 2 bezeichnet pA ein Poly (Adenylierungs)-Signal und prom einen Promotor. resist2 ist ein von resist verschiedenes Resistenzgen.

Die MHC I-Inaktivierung kann auch über andere virale Gene, z. B. das HSV-Gen ICP47, die CMV-Gene US6 und US3, die Adenovirusgene E3-19K sowie E6 oder das HIV NEF-Gen, den direkten Knock-out eines MHC I-Bestandteiles, wie z. B. des beta-2-Microglobulingens oder die interzelluläre Expression eines rekombinanten single chain Antikörpers zur Blockade der MHC 1-Präsentation auf der Zelloberfläche erfolgen.

Die Natürlichen Killerzellen können auch über einen monoklonalen Antikörper inhibiert werden, der die sogenannten inhibitory receptors der Natürlichen Killerzellen erkennt und die durch Natürliche Killerzellen vermittelte Zell-Lyse blockiert. Dieser monoklonale Antikörper kann auch als rekombinanter single chain Antikörper in der Membran der allogenen Spenderzelle verankert sein und dadurch angreifende Natürliche Killerzellen abwehren.

Die Herstellung eines membranständigen single chain Antikörpers ist prinzipiell beschrieben in Einfeld et al., "Construction of a pseudoreceptor that mediates transduction by adenoviruses expressing a ligand in fiber or penton base", J. Virol. Band 73, Seiten 9130-9136.

In einem ersten Schritt wird ein Hybridom hergestellt, das einen monoklonalen Antikörper produziert ; siehe z. B. Immunobiologie, 3. Ausgabe, Janeway et al., Current Biology Limited & Churchill Livingstone & Garland Publishing Inc., 1997. Das Hybridom ist die Fusion einer mortalen Antikörper-produzierenden Zelle aus der Milz einer immunisierten Maus mit einer immortalen Myelomzelle.

Die fusionierten Zellen haben die Eigenschaften beider Ausgangszellen, nämlich die Fähigkeit, Antikörper zu produzieren, sowie unsterblich zu sein. Die Hybridomzellen werden in einem speziellen Selektionsmedium (HAT-Medium) vermehrt und bezüglich der Expression des interessierenden Antikörpers analysiert.

Der nächste Schritt ist die Klonierung der Genabschnitte, die für die variablen Bereiche der leichten und schweren Kette des monoklonalen Antikörpers codieren. Über PCR mit Konsensus Primern innerhalb dieser variablen Bereiche werden die für die Antigenerkennung entscheidenden Gensequenzen amplifiziert. Die Gensequenz für die variable leichte Kette wird über einen kurzen Linker, der für ca. 15 Aminosäuren kodiert, mit der Gensequenz für die schwere Kette fusioniert. Dadurch wird das sogenannte Single Chain Fv (Fragment variable) erhalten.

Ein solcher in Zellen exprimierter single chain Antikörper kann über weitere Signal- und Verankerungssequenzen in der Zellmembran inseriert werden, so dass daraus der erfindungsgemäß verwendete membranständige single chain Antikörper resultiert.

Anstelle der von Einfeld et al. beschriebenen Erkennung eines linearen Hemagglutinin Epitops erkennt der erfindungsgemäße Antikörper jedoch ein Epitop auf natürlichen Killerzellen, wodurch die NK-vermittelte Zellyse blockiert wird. Das Gen für diesen single chain Antikörper kann statt des CMV-Genes UL18 in dem Genkomplex aus Fig. 2 enthalten sein.

Erfindungsgemäß können single chain Antikörper auch im Sinne von"Intrabodies"verwendet werden, so dass sie intrazelluläre Epitope erkennen und dadurch das Assembly oder den Transport von MHC 1-Molekülen blockieren. Das Gen für einen derartigen Antikörper kann ebenfalls in dem Genkomplex aus Fig. 2 vorhanden sein und bspw. die CMV-Gene US2 und US11 ersetzen.

Diese single chain Antikörper sind ausgewählt aus der Gruppe : Antikörper gegen TAP-Transporter (Anti-TAPI, Anti-TAPII), beta-2-Mikroglubolin, Calnexin, Calreticulin und Tapasin. Durch Expression und Aktivität eines oder mehrerer dieser intrazellulären single chain Antikörper wird die Präsentation von MHC I Molekülen auf der Oberfläche allogener Zellen verhindert und dadurch die Lyse durch zytotoxische T-Lymphozyten vermieden.

Der direkte Knock out eines MHC I-Bestandteiles beruht auf einem Verfahren, das ursprünglich für embryonale Stammzellen beschrieben wurde, um transgene Mäuse herzustellen. Eine allgemeine Übersicht über das"Ausknocken"von Genen findet sich in Koch-Brandt,"Gentransfer Prinzipien-Experimente-Anwendung bei Säugern", Thieme Verlag, 1993, sowie in Sedivy und Dutriaux,"Gene targeting and somatic cell genetics-a rebirth or a coming of age ?", Trends. Genet. Band 15, Seiten 88-90.

Bei diesem Verfahren wird ein für das auszuschaltende Gen homologer Bereich in ein Plasmid kloniert, wobei sich innerhalb dieses homologen Bereiches ein Resistenzgen und ausserhalb ein Suizidgen befindet. Das Plasmid wird in eine Zelle transfiziert, wobei der homologe Bereich in seltenen Fällen in das Zielgen integriert.

Durch das sich innerhalb des homologen Bereiches befindende Resistenzgen kann auf der einen Seite auf Integrationsereignisse selektioniert werden, andererseits wird dadurch die Expression des Zielgens auf dem betreffenden Allel unterbrochen. Da sich das Suizidgen ausserhalb des homologen Bereiches befindet, wird dieses nur bei einer illegitimen Rekombination mit integriert, nicht aber bei einer homologen Rekombination.

Mittels des Suizidgenes wird gegen Zellen selektioniert, bei denen eine illegitime Rekombination des Suizidgens stattgefunden hat. Mit diesem Verfahren wird also zunächst eines der beiden Allele eines Gens ausgeschaltet, im Falle der vorliegenden Erfindung z. B. das humane Gen für das beta-2-Mikroglobulin, das integraler Bestandteil des MHC I-Komplexes ist. Die Ausschaltung der beta-2-Mikroglobulin-Expression bewirkt somit die vollständige Abwesenheit von MHC 1-Molekülen auf der Zelloberfläche und verhindert somit die durch zytotoxische T-Lymphozyten vermittelte Zellyse.

Um auch das zweite Allel auszuschalten, muss die gleiche Strategie mit einem zweiten Resistenzgen verfolgt werden. Dieses kann über die identische homologe Sequenz in das zweite Allel integrieren. Auf dieses Ergebnis wird nun mit den beiden Resistenzen sowie mit dem Suizidgen selektioniert.

Die Herstellung vom beta-2-Mikroglobulin Knock out-Mäusen ist bereits beschrieben von Koller und Smithies,"Inactivating the beta 2-microglobulin locus in mouse embryonic stem cells by homologous recombination", PNAS, Band 86, Seiten 8932-8935. Für humane Zellen und die Anwendung solcher gegenüber zytotoxischen T-Lymphozyten resistenten Zellen zur Bereitstellung organbezogener allogener Zellen findet sich kein Vorbild in der Literatur.

Auf die oben beschriebene Weise können nicht nur Bestandteile des MHC I-Komplexes sondern auch eines oder beide Gene für den TAP-Transporter ausgeknockt werden, was ebenfalls die fehlende Präsentation von MHC 1-Molekülen auf der Zelloberfläche bewirkt. Knock out-Mäuse für TAP1 sind ebenfalls bekannt, siehe Behar et al.,"Susceptibility of mice deficient in CD1D or TAP1 to infection with Mycobacterium tuberculosis", J. Exp. Med. Band 189, Seiten 1973-1980.

### Beispiel 3 : Expansion und Differenzierung

Immortalisierte Ausgangszellen, die bereits terminal differenziert sind, werden in einem üblichen Medium expandiert, ohne dass weitere Maßnahmen erforderlich sind.

Handelt es sich bei den immortalisierten Zellen jedoch um mesenchymale Stammzellen aus dem Knochenmark, ist durch Zugabe von Differenzierungssubstanzen eine Differenzierung in die organspezifischen Stellen erforderlich.

Durch Behandlung mit 5'-Azacytidin können mesenchymale Stammzellen z. B. zu kardiomyogenen Zellen differenziert werden ; Makino et al. a. a. O. Eine 5'-Azacytidin-Behandlung von Stammzellen, die das Entwicklungspotential von Herzmuskelzellen haben, löst Differenzierungsvorgänge durch Demethylierung aus. Sehr wahrscheinlich wird dabei der Promotor von noch unbekannten essentiellen Herzmuskel-Differenzierungsgenen aktiviert.

Nach Erkenntnis der Erfinder hat 5'-Azacytidin aber ein mutagenes Potential. Daher wird die Differenzierung von Stammzellen zu Herzmuskelzellen erfindungsgemäß durch Gabe zumindest einer weiteren Differenzierungssubstanz verbessert. Hierzu ist die Substanz Trichstatin A (TSA) vorgesehen. TSA bewirkt eine Inhibition der Histon-Deacetylierung. Diese Histon-Deacetylierung steht im Zusammenhang mit transkriptioneller Repression von CpG-Methylierungen.

CpG-Inseln, also Bereiche mit mehreren CpG-Dinukleotiden, finden sich vorwiegend in Promotoren. Durch die Methylierungen kann die Aktivität eines CpG-reichen Promotors erheblich inhibiert werden. Dies erfolgt z. B. wenn 5'-Azacytidin in die DNA replizierender Zellen eingebaut wird, weil aufgrund der Aza-Gruppe an Position 5 dort keine Methylierung durch zelluläre Prozesse erfolgen kann.

Eine Kombination von 5'-Azacytidin und TSA kann somit synergistisch wirken, wie in Tumorzellen bereits gezeigt wurde ; siehe Cameron et al.,"Synergy of demethylation and histone deacetylase inhibition in the re-expression of genes silenced in cancer", Nat. Genet. Band 21, Seiten 103-107.

Dieser Synergismus wird erfindungsgemäß auf die Differenzierung von Stammzellen zu Herzmuskelzellen übertragen. Durch die weitere Zugabe von all-trans-Retinsäure und Amphotericin B wird die Differenzierung weiter optimiert. Retinsäure ist eine Differenzierungssubstanz, die in der Myoblasten-Zellinie H9C2 einen Herzmuskelphenotyp versus eines Skelettmuskelphenotyps begünstigt ; siehe Menard et al.,"Modulation of L-type calcium channel expression during retinoic acid-induced differentiation of H9C2 cardiac cells", J. Biol. Chem. Band 274, Seiten 2906329070.

Auch Amphotericin B kann die Differenzierung in Richtung Herzmuskelzellen günstig beeinflussen ; siehe Phinney et al. "Plastic adherent stromal cells from the bone marrow of commonly used strains of inbred mice : variations in yield, growth, and differentiation", J. Cell. Biochem. Band 72, Seiten 570-585.

Der Vorteil der Verwendung einer Kombination aus mehreren Differenzierungssubstanzen besteht darin, dass synergistische Effekte erzielt werden, die die mutagene Wirkung von 5' Azacytidin erheblich reduzieren oder gar aufheben. Dies ist für die spätere klinische Anwendung der aus Stammzellen gewonnenen Herzmuskelzellen von ausschlaggebender Bedeutung.

Wenn die Differenzierungssubstanz Dexamethason, Conget et al. a. a. O. alleine oder in Kombination mit den oben beschrieben vier Differenzierungssubstanzen verwendet wird, können Stammzellen zu Knochen- und Knorpelzellen differenziert werden.

### Beispiel 4 : Reversion der Immortalisierung

Für die spätere Transplantation der gemäß Beispiel 3 expandierten und ggf. differenzierten Zellen ist es erforderlich, dass die Immortalisierung wieder aufgehoben wird, damit die transplantierten Zellen nicht zu Tumorzellen entarten.

Dies erfolgt dadurch, dass mit Hilfe des Enzyms Cre-Rekombinase aus dem Genkomplex gemäß Fig. 1 der Genbereich zwischen den beiden LoxP-Stellen exzisiert wird. Dies kann durch Infektion der Zellen mit einem rekombinanten Adenovirus (Ad-Prom-Cre) erfolgen, das die Cre-Rekombinase exprimiert. Auf diese Weise kann die Immortalisierung zu einem gewünschten Zeitpunkt wieder rückgängig gemacht werden.

Die exzisierten DNA-Sequenzen können in den expandierten Zellen auch nicht mehr exprimiert werden, denn die Promotoren fehlen, da sie in dem homolog rekombinierten Genkomplex aus Fig. 1 verbleiben. Auch die in der DNA der Zelle integrierten Promotoren können keine benachbarten zellulären Sequenzen in cis aktivieren, da die Promotoren von Poly (Adenylierungs)-Signalen eingerahmt sind.

Als weitere Sicherheitsstufe ist jedoch in den Genkomplex aus Fig. 1 noch das Suizidgen TK eingebaut. Zellen mit noch intaktem Genkomplex aus Fig. 1 exprimieren die Thymidinkinase und können durch Gabe von Ganciclovir selektiv abgetötet werden.

Alternativ zu der Infektion von Zellen mit Ad-Prom-Cre kann das Enzym Cre-Rekombinase auch als Fusionsprotein, z. B. als rekombinantes Cre-VP22 verabreicht werden. Dieses zellgängige Fusionsprotein wird in das Zellkulturmedium gegeben und diffundiert wegen der Fusion mit dem Voyager-Protein VP22 in die expandierten Zellen.

### Beispiel 5 : Transplantation

Nach erfolgter Reversion der Immortalisierung gemäß Beispiel 4 und entsprechender Qualitätskontrolle werden die Zellen mit konventionellen Techniken in die geschädigten Organe transplantiert, z. B. mit einer Spritze in das Organ injiziert. Dies kann wiederholt geschehen, da aufgrund der Immortalisierung Material in jeder gewünschten Menge zur Verfügung steht.

Ohne die Immortalisierung könnten aus einer Stammzelle nur ca. 5 x 10⁸ bis 1 x 10⁹, bei einem älteren Spender u. U. sogar nur noch 1 x 10⁶ Zellen entstehen. Dies würde für eine Regeneration wahrscheinlich zu wenig sein. Bei autologer Transplantation muss der Patient ferner so lange warten, bis sich die Zellen zur erforderlichen Zellzahl vermehrt haben. Bei allogener Transplantation kann dagegen jederzeit die gewünschte Zellzahl bereitgestellt werden.

In besonderen Notfällen ist es sinnvoll, erst eine allogene Transplantation durchzuführen, um später auf autologe Transplantation umzusteigen.

## Patentansprüche

1. Genkomplex zur reversiblen Immortalisierung von Zellen, mit einem immortalisierenden Genbereich, der zumindest ein Resistenzgen, ein Telomerase-Gen und ein Suizidgen aufweist,
zwei den immortalisierenden Genbereich flankierenden Sequenzen, die als Erkennungsstellen für homologe intramolekulare Rekombination fungieren, und
zwei dem immortalisierenden Genbereich und den Sequenzen vorangestellten, jeweils flankierende Promotoren.

2. Genkomplex nach Anspruch 1, **dadurch gekennzeichnet, dass** er zusätzlich ein Transformationsgen enthält.

3. Genkomplex nach Anspruch 1 oder 2, **dadurch gekennzeichnet, dass** das Suizidgen das Thymidinkinase-Gen ist.

4. Genkomplex nach Anspruch 2 oder 3, **dadurch gekennzeichnet, dass** das Transformationsgen ein Onkogen, vorzugsweise das Tumorantigen von SV40 ist.

5. Genkomplex nach einem der Ansprüche 1 bis 4, **dadurch gekennzeichnet, dass** die flankierenden Sequenzen LoxP-Stellen sind:

6. Verfahren zur Gewinnung von Zellen, mit den Schritten :
(a) Bereitstellen von organbezogenen Zellen,
(b) Immortalisieren der organbezogenen Zellen durch Transfer des Genkomplexes aus einem der Ansprüche 1 bis 5,
(c) Expansion der immortalisierten Zellen aus (b) und Reversion der Immortalisierung der expandierten Zellen.

7. Verfahren nach Anspruch 6, **dadurch gekennzeichnet, dass** als organbezogene Zellen multipotente Stammzellen, vorzugsweise mesenchymale Stromazellen des Knochenmarks, verwendet werden.

8. Verfahren nach Anspruch 7, **dadurch gekennzeichnet, dass** die Expansion der immortalisierten Stammzellen unter Zugabe zumindest einer Differenzierungssubstanz erfolgt, die eine Differenzierung der Stammzellen zu organspezifischen Zellen fördert.

9. Verfahren nach Anspruch 8, **dadurch gekennzeichnet, dass** die Differenzierungssubstanz ausgewählt ist aus der Gruppe: Dexamethason, 5'-Azacytidin, Trichstatin A, all-trans Retinsäure und Amphotericin B.

10. Verfahren nach Anspruch 9, **dadurch gekennzeichnet, dass** eine Kombination von zumindest zwei, vorzugsweise vier Differenzierungssubstanzen verwendet wird.

11. Verfahren nach Anspruch 6, **dadurch gekennzeichnet, dass** als organbezogene Zellen ruhende, terminal differenzierte Ausgangszellen des Organs verwendet werden.

12. Verfahren nach Anspruch 11, **dadurch gekennzeichnet, dass** die Ausgangszellen im Zusammenhang mit der Immortalisierung transformiert werden.

13. Verfahren nach Anspruch 6, **dadurch gekennzeichnet, dass** durch das Resistenzgen auf erfolgreichen Transfer selektioniert wird.

14. Verfahren nach einem der Ansprüche 6 bis 13, **dadurch gekennzeichnet, dass** als organbezogene Zellen autologe Zellen verwendet werden.

15. Verfahren nach einem der Ansprüche 6 bis 13, **dadurch gekennzeichnet, dass** als organbezogene Zellen allogene Zellen verwendet werden.

16. Verfahren nach Anspruch 15, **dadurch gekennzeichnet, dass** bei den allogenen Zellen eine Immuntoleranz erzeugt wird.

17. Verfahren nach Anspruch 16, **dadurch gekennzeichnet, dass** durch einen monoklonalen Antikörper eine Immunmodulation bewirkt wird, der die inhibitory receptors von Natürlichen Killerzellen erkennt und die durch Natürliche Killerzellen vermittelte Zell-Lyse blockiert.

18. Verfahren nach Anspruch 17, **dadurch gekennzeichnet, dass** durch Knock out zumindest eines Genes der allogenen Zellen, vorzugsweise für beta-2-Mikroglobulin oder für TAP Transporter, die MHC 1-Präsentation auf der Zelloberfläche blockiert wird.

19. Verfahren nach einem der Ansprüche 6 bis 18, **dadurch gekenntzeichnet, dass** die Reversion der Immortalisierung durch Exzision des immortalisierenden Genbereiches aus dem in den expandierten Zellen befindlichen Genkomplex erfolgt.

20. Verfahren nach Anspruch 19, **dadurch gekennzeichnet, dass** die Exzision mit dem Enzym Cre-Rekombinase erfolgt.

21. Verfahren nach Anspruch 20, **dadurch gekennzeichnet, dass** das Enzym Cre-Rekombinase als rekombinantes zellgängiges Fusionsprotein verabreicht wird.

22. Verfahren nach Anspruch 21, **dadurch gekenntzeichnet, dass** die Zellen mit einem rekombinanten Virus infiziert werden, das das Enzym Cre-Rekombinase exprimiert.

23. Verfahren nach einem der Ansprüche 19 bis 22, **dadurch gekennzeichnet, dass** durch das Suizidgen auf erfolgreiche Exzision selektioniert wird.

24. Zellen, hergestellt nach dem Verfahren nach einem der Ansprüche 6 bis 23.

25. Verwendung der Zellen aus Anspruch 24 zur Herstellung eines Transplantates für die Regeneration eines Organes.

26. Verwendung der Zellen aus Anspruch 24 zur Herstellung eines Arzneimittels zur Behandlung chronischer Erkrankungen.

27. Arzneimittel, enthaltend Zellen nach Anspruch 24 in therapeutisch wirksamer Menge.

28. Plasmid, das den Genkomplex nach einem der Ansprüche 1 bis 5 enthält.

29. Viraler Vektor, der den Genkomplex nach einem der Ansprüche 1 bis 5 enthält.

30. Transplantat, das Zellen nach Anspruch 24 enthält.

31. Kit, enthaltend den Genkomplex nach einem der Ansprüche 1 bis 5.

## Claims

1. A gene complex for reversibly immortalizing cells, comprising an immortalizing gene region which includes at least one resistance gene, a telomerase gene, and a suicide gene, two sequences which flank the immortalizing gene region and function as recognition sites for homologous intramolecular recombination, and two promoters which are provided upstream of and which flank the immortalizing gene region and the sequences.

2. The gene complex according to claim 1, **characterized in that** in addition it comprises a transforming gene.

3. The gene complex according to claim 1 or 2, **characterized in that** the suicide gene is the thymidine kinase gene.

4. The gene complex according to claim 2 or 3, **characterized in that** the transforming gene is an oncogene, preferably the SV40 tumor antigen.

5. A gene complex according to any one of claims 1 to 4, **characterized in that** the flanking sequences are Lox-P sites.

6. A method for obtaining cells, comprising the following steps:
(a) providing organ-related cells,
(b) immortalizing the organ-related cells by transfer of the gene complex from any one of claims 1 to 5,
(c) expanding the immortalized cells from (b) and reversing the immortalization of the expanded cells.

7. The method according to claim 6, **characterized in that** multipotent stem cells, preferably mesenchymal stromal cells of the bone marrow, are used as the organ-related cells.

8. The method according to claim 7, **characterized in that** the immortalized stem cells are expanded by adding at least one differentiation substance which promotes a differentiation of the stem cells into organ-specific cells.

9. The method according to claim 8, **characterized in that** the differentiation substance is selected from the group consisting of dexamethasone, 5'-azacytidine, Trichstatin A, all-trans retinoic acid, and amphotericin B.

10. The method according to claim 9, **characterized in that** a combination of at least two, preferably four, differentiation substances is used.

11. The method according to claim 6, **characterized in that** resting, terminally differentiated parent cells of the organ are used as the organ-related cells.

12. The method according to claim 11, **characterized in that** the parent cells are transformed in connection with the immortalization.

13. The method according to claim 6, **characterized in that** the resistance gene is used to select for successful transfer.

14. A method according to any one of claims 6 to 13, **characterized in that** autologous cells are used as the organ-related cells.

15. A method according to any one of claims 6 to 13, **characterized in that** allogenic cells are used as the organ-related cells.

16. The method according to claim 15, **characterized in that** immunotolerance is generated in the allogenic cells.

17. The method according to claim 16, **characterized in that** immunomodulation is effected by a monoclonal antibody which recognizes the inhibitory receptors of natural killer cells and blocks cell lysis mediated by natural killer cells.

18. The method according to claim 17, **characterized in that** MHC 1 presentation on the cell surface is blocked by knocking out at least one gene of the allogenic cells, preferably beta-2 microglobulin or TAP transporter.

19. A method according to any one of claims 6 to 18, **characterized in that** the immortalization is reversed by excising the immortalizing gene region from the gene complex present in the expanded cells.

20. The method according to claim 19, **characterized in that** the excision is carried out using the enzyme Cre recombinase.

21. The method according to claim 20, **characterized in that** the Cre recombinase enzyme is administered as a recombinant cell-penetrating fusion protein.

22. The method according to claim 21, **characterized in that** the cells are infected with a recombinant virus expressing the Cre recombinase enzyme.

23. A method according to any one of claims 19 to 22, **characterized in that** the suicide gene is used to select for successful excision.

24. Cells, produced using the method according to any one of claims 6 to 23.

25. Use of the cells of claim 24 for producing a transplant for regenerating an organ.

26. Use of the cells of claim 24 for producing a pharmaceutical drug for treating chronic diseases.

27. A pharmaceutical drug comprising cells according to claim 24 in a therapeutically effective quantity.

28. A plasmid, comprising the gene complex according to any one of claims 1 to 5.

29. A viral vector, comprising the gene complex according to any one of claims 1 to 5.

30. A transplant, comprising the cells according to claim 24.

31. A kit, comprising the gene complex according to any one of claims 1 to 5.

## Revendications

1. - Complexe génique pour une immortalisation réversible de cellules, avec un domaine génique d'immortalisation qui comprend au moins un gène de résistance, un gène de télomérase et un gène suicide,
deux séquences flanquant le domaine génique d'immortalisation, qui font office de sites de reconnaissance pour une recombinaison intramoléculaire homologue, et
deux promoteurs flanquant respectivement le domaine génique d'immortalisation et les séquences présentés ci-dessus.

2. - Complexe génique selon la revendication 1, **caractérisé par le fait qu'**il comprend en outre un gène de transformation.

3. - Complexe génique selon l'une des revendications 1 ou 2, **caractérisé par le fait que** le gène suicide est un gène de thymidine kinase.

4. - Complexe génique selon l'une des revendications 2 ou 3, **caractérisé par le fait que** le gène de transformation est un oncogène, de préférence l'antigène tumoral de SV40.

5. - Complexe génique selon l'une des revendications 1 à 4, **caractérisé par le fait que** les séquences flanquantes sont des sites LoxP.

6. - Procédé d'obtention de cellules, comprenant les étapes de :
(a) apport de cellules associées à un organe,
(b) immortalisation des cellules associées à un organe par transfert du complexe génique tel que défini à l'une des revendications 1 à 5,
(c) développement des cellules immortalisées selon (b) et inversion de l'immortalisation des cellules développées.

7. - Procédé selon la revendication 6, **caractérisé par le fait que** des cellules souche multipotentes, de préférence des cellules mésenchymateuses du stroma de la moelle osseuse sont utilisées comme cellules associées à un organe.

8. - Procédé selon la revendication 7, **caractérisé par le fait que** le développement des cellules souches immortalisées est obtenu en présence additionnelle d'au moins une substance de différenciation qui favorise une différenciation des cellules souches en des cellules spécifiques d'un organe.

9. - Procédé selon la revendication 8, **caractérisé par le fait que** la substance de différenciation est choisie parmi le groupe : dexaméthasone, 5'-azacytidine, tristatine A, acide rétinoïque tout-trans et amphotéricine B.

10. - Procédé selon la revendication 9, **caractérisé par le fait qu'**une combinaison d'au moins deux, de préférence quatre substances de différenciation est utilisée.

11. - Procédé selon la revendication 6, **caractérisé par le fait que**, comme cellules associées à un organe, sont utilisées des cellules de départ de l'organe différenciées de façon terminale.

12. - Procédé selon la revendication 11, **caractérisé par le fait que** les cellules de départ sont transformées en association avec l'immortalisation.

13. - Procédé selon la revendication 6, **caractérisé par le fait qu'**un transfert réussi est sélectionné par l'intermédiaire du gène de résistance.

14. - Procédé selon l'une des revendications 6 à 13, **caractérisé par le fait que** des cellules autologues sont utilisées en tant que cellules associées à un organe.

15. - Procédé selon l'une quelconque des revendications 6 à 13, **caractérisé par le fait que** des cellules allogéniques sont utilisées en tant que cellules associées à un organe.

16. - Procédé selon la revendication 15, **caractérisé par le fait qu'**une immunotolérance est engendrée par les cellules allogéniques.

17. - Procédé selon la revendication 16, **caractérisé par le fait qu'**une immunomodulation est obtenue par l'intermédiaire d'un anticorps monoclonal, lequel reconnaît des récepteurs inhibiteurs de cellules tueuses naturelles et bloque la lyse cellulaire à médiation par les cellules tueuses naturelles.

18. - Procédé selon la revendication 17, **caractérisé par le fait que** la présentation du CMH 1 sur la surface cellulaire est bloquée par inactivation d'au moins un gène des cellules allogéniques, de préférence bêta-2 microglobuline ou transporteur TAP.

19. - Procédé selon l'une des revendications 6 à 18, **caractérisé par le fait que** l'inversion de l'immortalisation est obtenue par excision du domaine génique d'immortalisation à partir du complexe génique situé dans les cellules développées.

20. - Procédé selon la revendication 19, **caractérisé par le fait que** l'excision est obtenue par l'intermédiaire d'une enzyme Cre-recombinase.

21. - Procédé selon la revendication 20, **caractérisé par le fait que** l'enzyme Cre-recombinase est administrée en tant que protéine de fusion recombinante apte à entrer dans les cellules.

22. - Procédé selon la revendication 21, **caractérisé par le fait que** les cellules sont infectées par un virus recombinant, qui exprime l'enzyme Cre-recombinase.

23. - Procédé selon l'une des revendications 19 à 22, **caractérisé par le fait qu'**une excision réussie est sélectionnée par l'intermédiaire du gène suicide.

24. - Cellules produites selon le procédé tel que défini à l'une des revendications 6 à 23.

25. - Utilisation des cellules telles que définies à la revendication 24 pour la production d'un greffon pour la régénération d'un organe.

26. - Utilisation des cellules telles que définies à la revendication 24 pour la production d'un médicament pour le traitement de maladies chroniques.

27. - Médicament comprenant des cellules telles que définies à la revendication 24 dans un ensemble thérapeutiquement efficace.

28. - Plasmide, qui comprend le complexe génique tel que défini à l'une des revendications 1 à 5.

29. - Vecteur viral, qui comprend le complexe génique tel que défini à l'une des revendications 1 à 5.

30. - Greffon, qui comprend des cellules telles que définies à la revendication 24.

31. - Kit comprenant le complexe génique tel que défini à l'une des revendications 1 à 5.
